# EUROPÄISCHE PATENTSCHRIFT

(11) **EP 0 581 955 B1**
(45) Veröffentlichungstag und Bekanntmachung des Hinweises auf die Patenterteilung: **16.10.1996**
(21) Anmeldenummer: 93917380.3
(22) Anmeldetag: 02.02.1993
(51) Int. Cl.: A61K 7/42, C07D 235/18

(54) **VERWENDUNG VON BENZIMIDAZOL-DERIVATEN ALS LICHTSCHUTZFILTER**
USE OF BENZIMIDAZOLE DERIVATIVES AS PROTECTIVE LIGHT FILTERS
UTILISATION DE DERIVES DE BENZIMIDAZOL COMME SUBSTANCES FILTRANTES PROTECTRICES POUR L'EXPOSITION AUX RAYONS LUMINEUX

(30) Priorität: 13.02.1992 DE 4204257
(43) Veröffentlichungstag der Anmeldung: 09.02.1994
(73) Patentinhaber: MERCK PATENT GmbH, D-64271 Darmstadt (DE)
(72) Erfinder: STEIN, Ingeborg, D-6106 Erzhausen (DE); HEYWANG, Ulrich, D-6100 Darmstadt 14 (DE); PUTZ, Alexander, D-6120 Michelstadt (DE); MARTIN, Roland, D-6940 Weinheim (DE)
(86) Internationale Anmeldenummer: EP9300233
(87) Internationale Veröffentlichungsnummer: WO9315712

(56) Entgegenhaltungen:
- WO-A-92/21663
- CH-A- 350 763
- DE-A- 3 830 060
- FR-A- 1 279 273
- FR-A- 1 589 688
- GB-A- 1 004 073

## Beschreibung

Die Erfindung betrifft Benzimidazol-Derivate der Formel Ia, 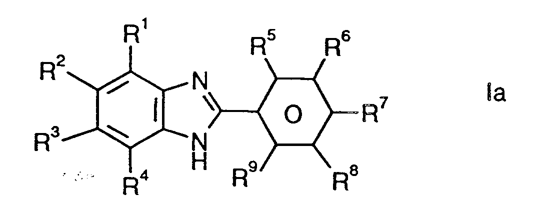 worin mindestens eine der Gruppen R¹ bis R⁹ eine 2-Ethylhexyloxygruppe ist und die anderen Gruppen R¹ bis R⁹ H, Alkyl oder Alkoxy mit 1 bis 10 C-Atomen bedeuten, sowie deren Verwendung als Lichtschutzfilter in kosmetischen Zubereitungen.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen; sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Aus dem deutschen Reichspatent Nr. 676/03 ist bekannt, daß ähnliche Benzimidazol-Derivate als Strahlenschutzmittel verwendet werden können, jedoch weisen diese dort beschriebenen Verbindungen lediglich Methyl- oder Methoxysubstituenten auf. Die gemäß der Erfindung als Lichtschutzfilter zu verwendeten sind teilweise bekannt (z.B. DE-OS 35 33 308) und teilweise neu.

Aus der WO-A-92/21663, das einen Stand der Technik nach A54(3) darstellt, und der FR-A-1279273 sind substituierte Phenylbenzimidazole bekannt, die allerdings als Substituent keinen 2-Ethylhexyloxyrest aufweisen.

Es wurde gefunden, daß 2-Phenylbenzimidazol-Derivate, welche eine 2-Ethylhexyloxygruppe besitzen, hervorragende UVB- bzw. UVA-B-Filter-Eigenschaften aufweisen. Ihre Löslichkeit in den in der Kosmetik verwendeten Ölen, insbesondere Paraffinöl, Neutralöl oder Isopropylmyristat, ist sehr gut, so daß Einsatzkonzentrationen auch in komplizierten Formulierungen bis mindestens 10 % der Zubereitung möglich sind.

Falls die Extinktion im UVA-Bereich ein Minimum aufweist, ist dies kein Nachteil, da man problemlos einen UVA-Filter mit in die Formulierung einarbeiten kann.

Außer ihren guten Eigenschaften als Filter zeichnen sich die erfindungsgemäßen Verbindungen durch eine gute thermische und photochemische Stabiltät aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung sind die Verbindungen der Formel Ia sowie deren Verwendung als Lichtschutzfilter in kosmetischen Zubereitungen.

Ein weiterer Gegenstand der Erfindung sind kosmetische Zubereitungen, welche als Lichtschutzfilter eine wirksame Menge mindestens einer Verbindung der Formel Ia, worin mindestens einer der Reste R¹ bis R⁹ 2-Ethylhexyloxy bedeutet, in einem kosmetisch verträglichen Träger enthält, insbesondere kosmetische Zubereitungen, welche 0,5 bis 10 Gew.% mindestens einer Verbindung der Formel Ia enthalten.

Ein weiterer Gegenstand der Erfindung sind kosmetische Zubereitungen, welche zusätzlich einen UV-B-Filter oder UV-A-Filter enthalten.

Von den neuen Verbindungen der Formel Ia, worin mindestens einer der Reste R¹ bis R⁹ 2-Ethylhexyloxy bedeutet, sind diejenigen der Formel Ia1 besonders bevorzugt: 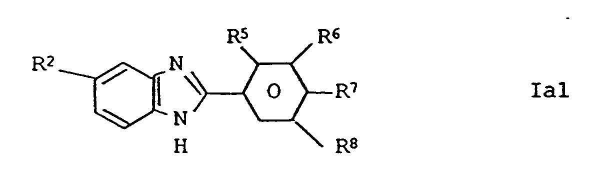 worin
- R²: H oder Alkyl mit 1 bis 6 C-Atomen,
einer oder zwei der Reste R⁵ bis R⁸ 2-Ethylhexyloxy und die anderen H oder Methoxy bedeuten.

In den Verbindungen der Formel Ia bedeuten R¹ bis R⁴ H, Alkyl und Alkoxy mit 1 bis 10 C-Atomen, vorzugsweise H oder unverzweigte Alkyl oder Alkoxygruppen mit 1 bis 8, insbesondere mit 1 bis 6 C-Atomen oder eine verzweigte Alkyl- oder Alkoxygruppe mit 3 bis 8, insbesondere mit 4 bis 8 C-Atomen, besonders bevorzugt ist die 2-Ethylhexyloxygruppe.

Vorzugsweise bedeuten drei der Reste R¹ bis R⁴, vorzugsweise R¹, R³ und R⁴ H und einer vorzugsweise R² H oder n-Alkyl mit 1 bis 6 C-Atomen, insbesondere Methyl.

Bevorzugte Verbindungen der Formel Ia sind diejenigen der Formeln Ia2 und Ia3, 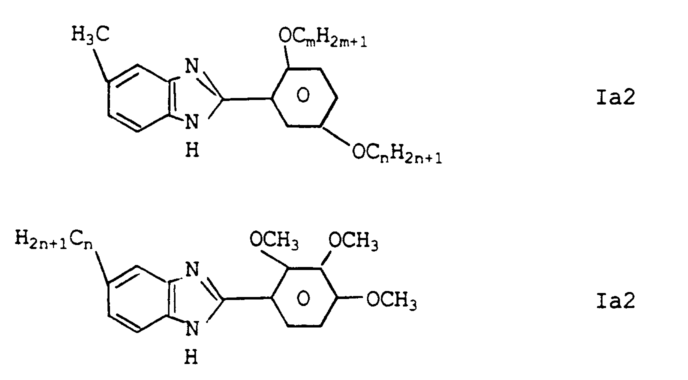 worin
- m und n: jeweils unabhängig voneinander eine ganze Zahl von 4 bis 10, vorzugsweise von 6 bis 8 bedeuten.

Die Herstellung der Verbindungen der Formel I erfolgt in Analogie zu bekannten Verfahren (z.B. nach DE-OS 35 33 308 oder P.N. Preston, Chemical Reviews Vol. 74, Nr. 3 (1974), S. 279-311), insbesondere jedoch durch Umsetzung der entsprechenden Arylaldehyde der Formel

Ar-CHO

mit den entsprechenden o-Phenylendiaminen der Formel 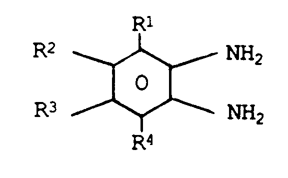 in Gegenwart von z.B Kupfer(II)acetat oder Natriumdisulfit.

### 1. Verfahren

Das o-Phenylendiamin wird vorzugsweise in einem protischen Lösungsmittel, insbesondere einem Alkohol, wie z.B. Methanol, Ethanol oder Isopropanol oder Wasser oder einem Gemisch dieser genannten Lösungsmittel zu der Kupfer(II)verbindung gegeben. Zu diesem Gemisch gibt man den Arylaldehyd in der Regel in einem protischen Lösungsmittel hinzu und erwärmt vorzugsweise bis zum Siedepunkt des Lösungsmittels.

### 2. Verfahren

Der entsprechende Arylaldehyd wird vorzugsweise in einem protischen Lösungsmittel mit Natriumbisulfit umgesetzt. Zu dem dabei entstehenden Bisulfit-Addukt wird das entsprechende o-Phenylendiamin gegeben.

Nach Erwärmen, vorzugsweise zum Siedepunkt des Lösungsmittels, wird das Gemisch in üblicher Weise aufgearbeitet.

Das erfindungsgemäße kosmetische Mittel wird als Mittel zum Schutz der menschlichen Epidermis oder der Haare oder als Sonnenschutzmittel verwendet.

Gegenstand der Erfindung ist ferner ein Verfahren zum Schutz der Haut und natürlicher oder sensibilisierter Haare von Sonnenstrahlen, wobei auf die Haut oder die Haare eine wirksame Menge mindestens einer Verbindung der Formel Ia, insbesondere worin mindestens einer der Reste R¹ bis R⁹ 2-Ethylhexyloxy bedeutet, aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzimidazol-Derivates der obigen Formel I, vorzugsweise der Formel Ia, insbesondere worin mindestens einer der Reste R¹ bis R⁹ 2-Ethylhexyloxy bedeutet, umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, ölig-wäßriger oder wäßrig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel Ia, worin mindestens einer der Reste R¹ bis R⁹ 2-Ethylhexyloxy bedeutet, ist in der Regel in einer Menge von 0,5 bis 10 %, vorzugsweise 1 bis 8 %, insbesondere 3 bis 6 %, bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel Ia, worin mindestens einer der Reste R¹ bis R⁹ 2-Ethylhexyloxy bedeutet, Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel Ia, insbesondere worin einer der Reste R¹ bis R⁹ 2-Ethylhexyloxy bedeutet, enthalten und andere UVB- und/oder UVA-Filter umfassen können.

In diesem Fall beträgt die Menge des Filters der Formel Ia in der Regel zwischen 0,5 und 8,0 Gew.%, vorzugsweise 1 bis 6 Gew.%, insbesondere 3 bis 5 Gew.%, bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shamponieren, vor oder nach dem Farben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflachenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel Ia, insbesondere worin einer der Reste R¹ bis R⁹ 2-Ethylhexyloxy bedeutet.

Die vorliegende Erfindung befaßt sich auch mit kosmetischen Mitteln, die mindestens eine Verbindung der Formel Ia, worin einer der Reste R¹ bis R⁹ 2-Ethylhexyloxy bedeutet, als Mittel zum Schutz vor UV-Strahlen und als Antioxidationsmittel enthalten; diese Mittel umfassen Haarprodukte, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel 1,0 bis 5,0 Gew.% einer Verbindung der Formel Ia, insbesondere worin einer der Reste R¹ bis R⁹ 2-Ethylhexyloxy bedeutet.

Ein weiterer Gegenstand der Erfindung ist die Verwendung der Verbindungen der Formel I als Sonnenfilter von großer Absorptionsbreite in einem Wellenlängenbereich von 300 bis 350 nm.

Die Verbindungen der Formel Ia, worin mindestens einer der Reste R¹ bis R⁹ 2-Ethylhexyloxy bedeutet, zeigen eine signifikante pharmakologische Aktivität auf dem Gebiet der Präventivbehandlung von Entzündungen und Hautallergien.

Die Verbindungen der Formel Ia, worin mindestens einer der Reste R¹ bis R⁹ 2-Ethylhexyloxy bedeutet, zur Verwendung als Medikament geeignet.

Bevorzugt ist ferner ein pharmazeutisches Mittel, welches eine wirksame Menge mindestens einer Verbindung der Formel Ia, worin mindestens einer der Reste R¹ bis R⁹ 2-Ethylhexyloxy bedeutet, als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanaesthetika, Puffer etc. enthalten.

Es kann außerdem in an sich bekannter Weise in Retard-Form oder; in einer Form konditioniert sein, in welcher der Wirkstoff rasch freigesetzt wird.

Auch ohne weitere Ausführungen wird davon ausgegangen, daß ein Fachmann die obige Beschreibung in weitesten Umfang nutzen kann Die bevorzugten Ausführungsformen sind deswegen lediglich als beschreibende, keineswegs als in irgendeine Weise limitierende offenbarung aufzufassen.

Die nachfolgenden Beispiele sollen die Erfindung erläutern.

### Herstellungsbeispiele

Die UV-Spektren wurden in einem UV-Spektrometer von Perkin-Elmer bei einer Schichtdicke von einem Zentimeter gemessen.

### Beispiel 1

### 2-[2',5'-Di-(2-Ethyl-hexyloxy)-phenyl]-benzimidazol

35 g (0,17 mol) Kupfer(II)acetat in 600 ml Wasser werden zu einer Lösung von 5,4 g (0,05 mol) 1,2-Phenylendiamin in 200 ml Methanol gegeben und kurz nachgerührt. 19 g (0,05 mol) 2,5-Di-(2-Ethyl-hexyloxy)-benzaldehyd in 100 ml Methanol werden anschließend dazugegossen und die Mischung wird unter Rückfluß erhitzt. Danach wird heiß abfiltriert, Ethanol versetzt und unter Rückfluß wird durch diese Suspension H₂S-Gas durchgeleitet. Es wird heiß abgesaugt und die Lösung wird eingeengt. Die Aufreinigung erfolgt mittels Säulenchromatographie. Es entsteht ein Öl, dessen NMR-, MS- und IR-Spektren der erwarteten Struktur entsprechen.
- UV (Ethanol, c = 1 mg/100 ml):: λₘₐₓ₁ = 303 nm, E = 0,34
λₘₐₓ₂ = 328 nm, E = 0,30

### Analog werden hergestellt:

2-(4'-(2-Ethylhexyloxy)-phenyl)-benzimidazol, Fp = 158-159 °C
2-(3'-(2-Ethylhexyloxy)-phenyl)-benzimidazol, Fp = 140-141 °C
2-(2'-(2-Ethylhexyloxy)-phenyl)-benzimidazol, Fp = 63-65 °C
2-(3'-Methoxy-4'-(2-Ethylhexyloxy)-phenyl)-benzimidazol, Öl
2-(4'-Methoxy-3'-(2-ethylhexyloxy)-phenyl)-benzimidazol, Öl
2-(2',5'-Di(2-ethylhexyloxy)-phenyl)-5-n-pentylbenzimidazol, Öl.

Verbindungen der Formel Ia2 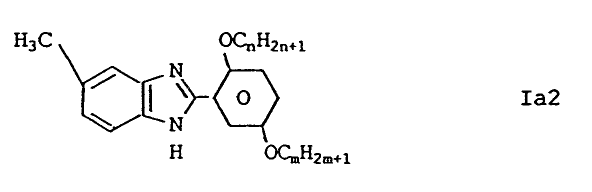
2-[2',5'-Di(2-ethylhexyloxy)-phenyl]-5-methyl-benzimidazol, Fp -62 bis -60 °C (Öl).

### Beispiel 2

### 2-(2',4'-Dimethoxy-3'-(2-Ethyl-hexyloxy))-phenyl-benzimidazol

a) 2-(2',3',4'-Trimethoxy)-phenyl-benzimidazol
70 g (0,36 mol) Natriumdisulfit in 800 ml Wasser werden mit 69 g (0,35 mol) 2,3,4-Trimethoxy-benzaldehyd versetzt und kurz nachgerührt. Zu dieser Reaktionsmischung werden 35 g (0,32 mol) Phenylendiamin gegebne und unter Rückfluß erhitzt. Das entstandene Öl wird abgetrennt und aus Toluol/Petrolether auskristallisiert. Es entstehen 62 g (68 % d. Th.) weiße Kristalle. Fp.: 167 °C
- UV (Ethanol, c = 1 mg/100 ml):: λₘₐₓ₁ = 309 nm, E = 0,76
λₘₐₓ₂ = 323 nm, E = 0,42

b) 2-(2',4'-Dimethoxy-3'-(2-ethyl-hexyloxy))-phenyl-benzimidazol
Zu den 44 g (0,16 mol) nach Vorschrift b) erhaltenen 2-(2',4'-Dimethoxy-3'-hydroxy)-phenyl-benzimidazol in 400 ml Dimethylformamid werden 47 g (0,34 mol) Kaliumcarbonat und 16 g (0,12 mol) Lithiumiodid gegeben. Unter Rühren werden 50 g (0,34 mol) 2-Ethyl-hexylchlorid zugetropft und die Reaktionslösung wird am Rückfluß gekocht. Nach Reaktionsende wird die Lösung auf Wasser gegossen, die organische Phase wird abgetrennt und eingeengt. Die Aufreinigung erfolgt mittels Säulenchromatographie. Es entstehen 26 g eines Öls (42 % d. Th.). Die Spektren entsprechen der erwarteten Verbindung.
- UV (Ethanol, c = 1 mg/100 ml):: λₘₐₓ₁ = 297 nm, E = 0,51
λₘₐₓ₂ = 308 nm, E = 0,45

| Löslichkeit: | |
|---|---|
| Paraffin: | 5,7 g/100 ml |
| Miglyol: | > 59 g/100 ml |
| Isopropanol: | > 64 g/100 ml |

### Anwendungsbeispiele

In den folgenden Beispielen werden die einzelnen Komponenten wie angegeben, gegebenenfalls bei erhöhter Temperatur miteinander gemischt und homogenisiert Anschließend wird das Gemisch, gegebenenfalls unter Rühren abgekühlt und, falls erwünscht, bei 40 °C parfümiert.

### Beispiel A

| Sonnenschutzcreme (W/O) | | | % |
|---|---|---|---|
| A | 2-[2',5'-Di(2-ethylhexyloxy)-phenyl]-benzimidazol (aus Beispiel 1) | (1) | 4,00 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s. |
| | Wasser, demineralisiert | ad | 100,00 |

### Herstellung:

Phase A wird auf 75 °C, Phase B auf 80 °C erhitzt. Phase B wird langsam in Phase A eingerührt.

### Bezugsquellen:

(1) E. Merck, Darmstadt
(2) ICI, Essen
(3) Hüls Troisdorf AG, Witten
(4) Dow Corning, Düsseldorf

### Beispiel B

| Sonnenschutzcreme (W/O) | | | % |
|---|---|---|---|
| A | 2-[2',5'-Di(2-ethylhexyloxy)-phenyl]-benzimidazol (aus Beispiel 1) | (1) | 6,00 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s. |
| | Wasser, demineralisiert | ad | 100,00 |

### Herstellung:

Phase A wird auf 75 °C, Phase B auf 80 °C erhitzt. Phase B wird langsam in Phase A eingerührt.

### Bezugsquellen:

(1) E. Merck, Darmstadt
(2) ICI, Essen
(3) Hüls Troisdorf AG, Witten
(4) Dow Corning, Düsseldorf

### Beispiel C

| Sonnenschutzgel (ölhaltig) | | % |
|---|---|---|
| 2-[2',5'-Di(2-ethylhexyloxy)-phenyl]-benzimidazol (aus Beispiel 1) | (1) | 5,00 |
| Paraffinöl flüssig (Art.-Nr. 7162) | (1) | 20,00 |
| Miglyol 812 | (2) | 15,00 |
| Isopropylmyristat | (3) | 5,00 |
| Vaseline | (4) | 55,00 |

### Herstellung:

Zum Lösen werden alle Bestandteile auf 75 °C erwärmt.

### Bezugsquellen:

(1) E. Merck, Darmstadt
(2) Hüls Troisdorf AG, Witten
(3) Henkel, Düsseldorf
(4) E. Wagner, Bremen

### Beispiel D

| Hautpflegecreme (O/W) mit Lanolin | | | % |
|---|---|---|---|
| A | 2-[2',5'-Di(2-ethylhexyloxy)-phenyl]-benzimidazol (aus Beispiel 1) | (1) | 4,00 |
| | Cutina KD 16 | (2) | 3,00 |
| | Stearinsäure (Art.-Nr. 671) | (1) | 2,00 |
| | Lanolin Corona | (3) | 3,00 |
| | Antaron V-220 | (4) | 1,00 |
| | Oxynex 2004 (Art.-Nr. 6940) | (1) | 0,05 |
| B | Tris(hydroxymethyl)aminomethan (Art.-Nr. 8386) | (1) | 0,81 |
| | Karion F flüssig (Art.-Nr. 2993) | (1) | 5,00 |
| | Carbopol 940 | (5) | 0,05 |
| | Konservierungsmittel | | q.s. |
| | Wasser, demineralisiert | ad | 100,00 |

### Herstellung:

Phase B wird kurz homogenisiert. Phase A wird auf 75 °C, Phase B auf 80 °C erhitzt. Phase B wird langsam in Phase A eingerührt.

### Bezugsquellen:

(1) E. Merck, Darmstadt
(2) Henkel, Düsseldorf
(3) Croda, Nettetal
(4) GAF, Frechen
(5) Goodrich, Neuss

### Beispiel E

| Hautpflegecreme (O/W) mit Lanolin | | | % |
|---|---|---|---|
| A | 2-(2',5'-Di(2-ethylhexyloxy)-phenyl)-benzimidazol (aus Beispiel 1) | (1) | 6,00 |
| | Cutina KD 16 | (2) | 3,00 |
| | Stearinsäure (Art.-Nr. 671) | (1) | 2,00 |
| | Lanolin Corona | (3) | 3,00 |
| | Antaron V-220 | (4) | 1,00 |
| | Oxynex 2004 (Art.-Nr. 6940) | (1) | 0,05 |
| B | Tris(hydroxymethyl)aminomethan (Art.-Nr. 8386) | (1) | 0,81 |
| | Karion F flüssig (Art.-Nr. 2993) | (1) | 5,00 |
| | Carbopol 940 | (5) | 0,05 |
| | Konservierungsmittel | | q.s. |
| | Wasser, demineralisiert | ad | 100,00 |

### Herstellung:

Phase B wird kurz homogenisiert. Phase A wird auf 75 °C, Phase B auf 80 °C erhitzt. Phase B wird langsam in Phase A eingerührt.

### Bezugsquellen:

(1) E. Merck, Darmstadt
(2) Henkel, Düsseldorf
(3) Croda, Nettetal
(4) GAF, Frechen
(5) Goodrich, Neuss

### Beispiel F

| Sonnenschutzöl | | % |
|---|---|---|
| 2-[2',5'-Di(2-ethylhexyloxy)-phenyl]-benzimidazol (aus Beispiel 1) | (1) | 5,00 |
| Paraffinöl dünnflüssig (Art.-Nr. 7174) | (1) | 50,00 |
| Miglyol 812 | (2) | 15,00 |
| Cetiol B | (3) | 22,50 |
| Isopropylmyristat | (3) | 7,50 |

### Herstellung:

Man erhitzt unter Rühren auf 70 °C bis alle Komponenten gelöst sind.

### Bezugsquellen:

(1) E. Merck, Darmstadt
(2) Hüls Troisdorf AG, Witten
(3) Henkel, Düsseldorf

### Beispiel G

| Sonnenschutzcreme (W/O) | | | % |
|---|---|---|---|
| A | 2-[2',4'-Dimethoxy-3'-(2-ethylhexyloxy)-phenyl]-benzimidazol (aus Beispiel 2) | (1) | 4,00 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s. |
| | Wasser, demineralisiert | ad | 100,00 |

### Herstellung:

Phase A wird auf 75 °C, Phase B auf 80 °C erhitzt. Phase B wird langsam in Phase A eingerührt.

### Bezugsquellen:

(1) E. Merck, Darmstadt
(2) ICI, Essen
(3) Hüls Troisdorf AG, Witten
(4) Dow Corning, Düsseldorf

### Beispiel H

| Sonnenschutzcreme (W/O) | | | % |
|---|---|---|---|
| A | 2-[2',4'-Dimethoxy-3'-(2-ethylhexyloxy)-phenyl]-benzimidazol (aus Beispiel 2) | (1) | 6,00 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s. |
| | Wasser, demineralisiert | ad | 100,00 |

### Herstellung:

Phase A wird auf 75 °C, Phase B auf 80 °C erhitzt. Phase B wird langsam in Phase A eingerührt.

### Bezugsquellen:

(1) E. Merck, Darmstadt
(2) ICI, Essen
(3) Hüls Troisdorf AG, Witten
(4) Dow Corning, Düsseldorf

### Beispiel I

| Sonnenschutzöl | | % |
|---|---|---|
| 2-[2',4'-Dimethoxy-3'-(2-ethylhexyloxy)-phenyl]-benzimidazol (aus Beispiel 2) | (1) | 5,00 |
| Paraffinöl dünnflüssig (Art.-Nr. 7174 | (1) | 50,00 |
| Miglyol 812 | (2) | 15,00 |
| Cetiol B | (3) | 22,50 |
| Isopropylmyristat | (3) | 7,50 |

### Herstellung:

Man erhitzt unter Rühren auf 70 °C bis alle Komponenten gelöst sind.

### Bezugsquellen:

(1) E. Merck, Darmstadt
(2) Hüls Troisdorf AG, Witten
(3) Henkel, Düsseldorf

### Beispiel J

| Hautpflegecreme (O/W) mit Lanolin | | | % |
|---|---|---|---|
| A | 2-[2',4'-Dimethoxy-3'-(2-ethylhexyloxy)-phenyl]-benzimidazol (aus Beispiel 2) | (1) | 4,00 |
| | Cutina KD 16 | (2) | 3,00 |
| | Stearinsäure (Art.-Nr. 671) | (1) | 2,00 |
| | Lanolin Corona | (3) | 3,00 |
| | Antaron V-220 | (4) | 1,00 |
| | Oxynex 2004 (Art.-Nr. 6940) | (1) | 0,05 |
| B | Tris(hydroxymethyl)aminomethan (Art.-Nr. 8386) | (1) | 0,81 |
| | Karion F flüssig (Art.-Nr. 2993) | (1) | 5,00 |
| | Carbopol 940 | (5) | 0,05 |
| | Konservierungsmittel | | q.s. |
| | Wasser, demineralisiert | ad | 100,00 |

### Herstellung:

Phase B wird kurz homogenisiert. Phase A wird auf 75 °C, Phase B auf 80 °C erhitzt. Phase B wird langsam in Phase A eingerührt.

### Bezugsquellen:

(1) E. Merck, Darmstadt
(2) Henkel, Düsseldorf
(3) Croda, Nettetal
(4) GAF, Frechen
(5) Goodrich, Neuss

### Beispiel K

| Hautpflegecreme (O/W) mit Lanolin | | | % |
|---|---|---|---|
| A | 2-[2',4'-Dimethoxy-3'-(2-ethylhexyloxy)-phenyl]-benzimidazol (aus Beispiel 2) | (1) | 6,00 |
| | Cutina KD 16 | (2) | 3,00 |
| | Stearinsäure (Art.-Nr. 671) | (1) | 2,00 |
| | Lanolin Corona | (3) | 3,00 |
| | Antaron V-220 | (4) | 1,00 |
| | Oxynex 2004 (Art.-Nr. 6940) | (1) | 0,05 |
| B | Tris(hydroxymethyl)aminomethan (Art.-Nr. 8386) | (1) | 0,81 |
| | Karion F flüssig (Art.-Nr. 2993) | (1) | 5,00 |
| | Carbopol 940 | (5) | 0,05 |
| | Konservierungsmittel | | q.s. |
| | Wasser, demineralisiert | ad | 100,00 |

### Herstellung:

Phase B wird kurz homogenisiert. Phase A wird auf 75 °C, Phase B auf 80 °C erhitzt. Phase B wird langsam in Phase A eingerührt.

### Bezugsquellen:

(1) E. Merck, Darmstadt
(2) Henkel, Düsseldorf
(3) Croda, Nettetal
(4) GAF, Frechen
(5) Goodrich, Neuss

### Beispiel L

| Sonnenschutzgel (ölhaltig) | | % |
|---|---|---|
| 2-(2',4'-Dimethoxy-3'-(2-ethylhexyloxy)-phenyl)-benzimidazol (aus Beispiel 2) | (1) | 5,00 |
| Paraffinöl flüssig (Art.-Nr. 7162) | (1) | 20,00 |
| Miglyol 812 | (2) | 15,00 |
| Isopropylmyristat | (3) | 5,00 |
| Vaseline | (4) | 55,00 |

### Herstellung:

Zum Lösen werden alle Bestandteile auf 75 °C erwärmt.

### Bezugsquellen:

(1) E. Merck, Darmstadt
(2) Hüls Troisdorf AG, Witten
(3) Henkel, Düsseldorf
(4) E. Wagner, Bremen

## Patentansprüche

1. Benzimidazole der Formel Ia, 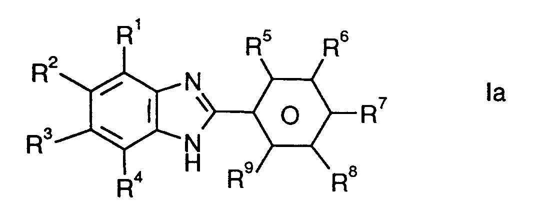 worin mindestens eine der Gruppen R¹ bis R⁹ eine 2-Ethylhexyloxygruppe ist und die anderen Gruppen R¹ bis R⁹ H, Alkyl oder Alkoxy mit 1 bis 10 C-Atomen bedeuten.

2. Verwendung von Benzimidazolen der Formel Ia nach Anspruch 1, 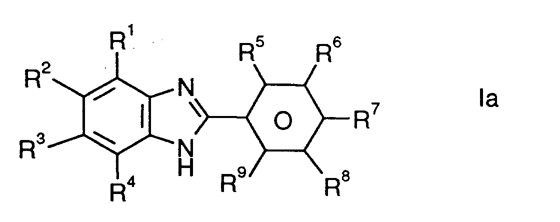 worin mindestens einer der Gruppen R¹ bis R⁹ eine 2-Ethylhexyloxygruppe ist und die anderen Gruppen R¹ bis R⁹ H, Alkyl oder Alkoxy mit 1 bis 10 C-Atomen bedeuten, als Lichtschutzfilter in kosmetischen Zubereitungen.

3. Kosmetische Zubereitung, dadurch gekennzeichnet, daß sie als Lichtschutzfilter eine wirksame Menge mindestens einer Verbindung der Formel Ia nach Anspruch 1 in einem kosmetisch verträglichen Träger enthält.

4. Kosmetische Zubereitung nach Anspruch 3, wobei der Träger mindestens eine Fettphase aufweist, dadurch gekennzeichnet, daß sie mindestens eine Verbindung der Formel Ia enthält.

5. Kosmetische Zubereitung nach einem der Ansprüche 3 bis 4, dadurch gekennzeichnet, daß sie 0,5 bis 10 Gew.% mindestens einer Verbindung der Formel Ia enthält.

6. Kosmetische Zubereitung nach einem der Ansprüche 3 bis 5, dadurch gekennzeichnet, daß sie zusätzlich einen UV-A-Filter oder UV-B-Filter enthält.

7. Kosmetische Verwendung der Verbindungen der Formel Ia nach Anspruch 1 als Sonnenfilter in einem Wellenlängenbereich von 300 bis 350 nm.

## Claims

1. Benzimidazoles of the formula Ia 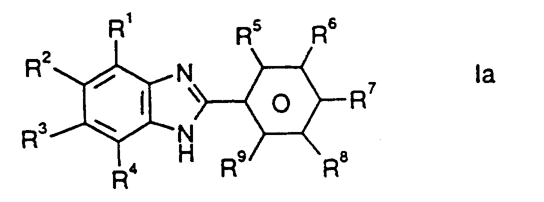 wherein at least one of the groups R¹ to R⁹ is a 2-ethyl-hexyloxy group and the other groups R¹ to R⁹ are H, alkyl or alkoxy having 1 to 10 C atoms.

2. Use of benzimidazoles of the formula Ia according to Claim 1 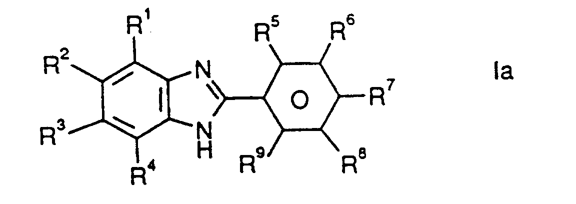 wherein at least one of the groups R¹ to R⁹ is a 2-ethyl-hexyloxy group and the other groups R¹ to R⁹ are H, alkyl or alkoxy having 1 to 10 C atoms, as light protection filters in cosmetic formulations.

3. Cosmetic formulation, characterized in that it comprises, as a light protection filter, an active amount of at least one compound of the formula Ia according to Claim 1 in a cosmetically tolerated carrier.

4. Cosmetic formulation according to Claim 3, wherein the carrier has at least one fatty phase, characterized in that this comprises at least one compound of the formula Ia.

5. Cosmetic formulation according to one of Claims 3 to 4, characterized in that it comprises 0.5 to 10% by weight of at least one compound of the formula Ia.

6. Cosmetic formulation according to one of Claims 3 to 5, characterized in that it additionally comprises a UV-A filter or UV-B filter.

7. Cosmetic use of the compounds of the formula Ia according to Claim 1 as solar filters in a wavelength range from 300 to 350 nm.

## Revendications

1. Benzimidazoles de formule Ia, 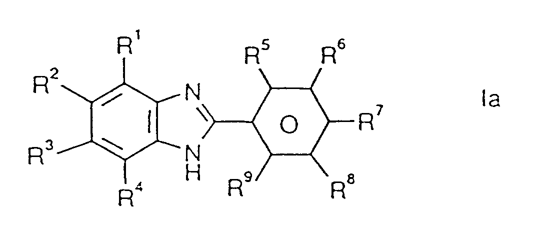 dans laquelle au moins un des groupes R¹ à R⁹ est un groupe 2-éthylhexyloxy et les autres groupes R¹ à R⁹ représentent H, un alkyle ou un alcoxy en C₁ à C₁₀.

2. Utilisation de benzimidazoles de formule Ia selon la revendication 1, 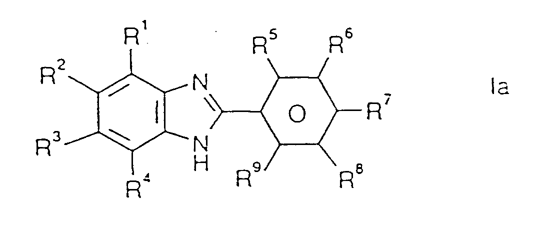 dans laquelle au moins un des groupes R¹ à R⁹ est un groupe 2-éthylhexyloxy et les autres groupes R¹ à R⁹ représentent H, un alkyle ou un alcoxy en C₁ à C₁₀ comme flitre protecteur contre la lumière dans les préparations cosmétiques.

3. Préparation cosmétique caractérisée en ce qu'elle contient comme filtre protecteur contre la lumière une quantité efficace d'au moins un composé de formule Ia selon la revendication 1 dans un support cosmétiquement compatible.

4. Préparation cosmétique selon la revendication 3, dans laquelle le support présente au moins une phase grasse, caractérisée en ce qu'elle contient au moins un composé de formule Ia.

5. Préparation cosmétique selon l'une des revendications 3 à 4, caractérisée en ce qu'elle contient de 0,5 à 10% en poids d'au moins un composé de formule Ia.

6. Préparation cosmétique selon l'une des revendications 3 à 5, caractérisée en ce qu'elle contient en outre un filtre d'UV-A ou un filtre d'UV-B.

7. Utilisation cosmétique des composés de formule Ia selon la revendication 1 comme filtres solaires dans un intervalle de longueurs d'onde comp'is entre 300 et 350 nm.
